# EUROPEAN PATENT SPECIFICATION

(11) **EP 1 938 810 B1**
(45) Date of publication and mention of the grant of the patent: **18.06.2014**
(21) Application number: 06810942.0
(22) Date of filing: 29.09.2006
(51) Int. Cl.: A61K 31/122, A23L 1/30, A61K 36/02, A61P 3/04, A61P 3/06, A61P 3/10, A61P 5/50, A61P 9/12, A61P 43/00, A61K 36/23, A61K 36/38, A61K 36/489, A61K 36/61, A61K 36/734, A23L 1/337, A23L 2/52, A23G 1/32, A61K 36/48, A61K 36/53, A61K 36/73

(54) **AMELIORATING AGENT FOR METABOLIC SYNDROME**
MITTEL ZUR LINDERUNG DES METABOLISCHEN SYNDROMS
AGENT D'AMELIORATION POUR LE SYNDROME METABOLIQUE

(30) Priority: 30.09.2005 JP 2005289010
(43) Date of publication of application: 02.07.2008
(73) Proprietor: FUJI CHEMICAL INDUSTRY CO., LTD., Nakaniikawa-gun, Toyama 930-0397 (JP)
(72) Inventor: TAKAHASHI, Jiro, Nakaniikawa-gun Toyama; 9300397 (JP); HUSSEIN, Ghazi, Toyama-shi Toyama; 9398224 (JP); SANKAWA, Ushio, Toyama-shi Toyama; 9398224 (JP); GOTO, Hirozou, Toyama-shi Toyama; 9300194 (JP)
(74) Representative: Albrecht, Thomas
(86) International application number: PCT/JP2006/319587
(87) International publication number: WO 2007/037438

(56) References cited:
- WO-A1-2005/074907
- JP-A- 2003 064 360
- JP-A- 2003 335 668
- JP-A- 2006 016 408
- MARCH B E ET AL: "INTESTINAL ABSORPTION OF ASTAXANTHIN PLASMA ASTAXANTHIN CONCENTRATION BODY WEIGHT AND METABOLIC RATE AS DETERMINANTS OF FLESH PIGMENTATION IN SALMONID FISH", AQUACULTURE, ELSEVIER, AMSTERDAM, NL, vol. 90, no. 3-4, 1 November 1990 (1990-11-01), pages 313-322, XP008135369, ISSN: 0044-8486, DOI: DOI:10.1016/0044-8486(90)90255-L [retrieved on 2003-10-03]
- IKEUCHI M ET AL: "Effects of astaxanthin in obese mice fed a high-fat diet", BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN, vol. 71, no. 4, 1 January 2007 (2007-01-01), pages 893-899, XP008135383, ISSN: 0916-8451, DOI: DOI:10.1271/BBB.60521
- KUNITOMO M.: 'Metabolic Syndrome Model Rat ni Shojiru Sanka Stress to Ensho Han'no no Zoka: Vitamin E Toyo no Eikyo' FOLIA PHARMACOLOGICA JAPONICA vol. 124, no. SUPPL. 1, 01 November 2004, pages 37P - 38P, XP003011502
- HUSSEIN G. ET AL.: 'Astaxanthin ameliorates features of metabolic syndrome in rat model SHR/NDmcr-cp (cp/cp)' J. PHARMACOL. SCI. vol. 100, no. SUPPL. 1, 20 February 2006, page 176P, XP003011503

## Description

### Technical Field

The present invention relates to a composition for use in a method to ameliorate and/or prevent metabolic syndrome which contains astaxanthin as an effective ingredient. More particularly, it relates to a composition having an effect of ameliorating and/or preventing metabolic syndrome which contains astaxanthin as an effective ingredient, said astaxanthin being obtained by drying and pulverizing *Haetmatococcus* alga cultured in a hermetic type of cultivation apparatus, subjecting the pulverizied *Haetmatococcus* alga to extraction with a solvent and thereafter removing the solvent from the resultant extract. Also, it relates to a composition for use in a method of ameliorating and/or preventing metabolic syndrome which contains astaxanthin and one or more of active agents as an effective ingredient.

### Background Art

In Japan, circulatory system diseases such as derebrocascular accident and cardiovascular occupy about 30 percent of the total death rate, and become the highest cause of death together with cancer. Even though they do not lead to death, the heavy aftereffect accompanied by attack leaves and it becomes serious problem. It has been hitherto considered as the main cause of circulatory system diseases that symptoms such as hyperlipemia, hyperpiesia, hyperglycemia, etc. have individually developed. Although they become the cause singly, it has been recently identified that a combination of three or more of hypercholesterolemia, hypertriceridemia, impaired glucose tolerance, hypertension and obesity causes rather high attack rate that is several tens times than the single case, even though the individual symptoms are not serious. Multiple risk factor syndrome where their syndrome being slight gather in a person are called metabolic syndrome.

It is known that metabolic syndrome is due to the increase of mast cell by accumulation of fat in the enteric organ as the highest cause. The increase of mast cell causes abnormalities of various physiologically active substances which it secrets and the increase in the released free fatty acid. As the physiologically active substances, TNFα and resistin which are related to insulin resistance, PAI-1 which is related to the formation of thrombus, angiotensinogen which is related to hypertension and the like are taken.

With respect to hyperlipemia, hyperpiesia, hyperglycemia, etc. which are the individual symptoms of metabolic syndrome to be formed, there have been developed a number of medicines for treating the individual symptoms. In the case of pharmaceutical preparation, however, there are many ones which are not preferable for a long-term intake or preventative intake because of their side effect and a bad effect to be caused by the combination of different kinds of medicines (drug interaction). And therefore, a beverage, a food or a medicine for ameliorating and preventing metabolic syndrome have been desired which do not have a side effect and toxicity and which are edible in the forms of a beverage and a food which enable a long term intake.

Astaxanthin is a kind of the same carotenoid with β-carotene and widely distributed in natural origin especially in ocean as in crustaceans such as shrimp, crab, etc.; fishes such as a salmon, a porgy, etc.; algae such as green alga of Haematococcus, etc.; yeasts such as *Phaffia* red yeast, etc., and it has been widely as a red pigment. It has been found to have antioxidative action that is about 1000 times as strong as vitamin E (α-tocopherol) and about 40 times as strong as β-carotene. At the present time, it is being used as the most effective healthy food.

As the main functional characteristics which astaxanthin has, its preventative effect on arteriosclerosis (Patent literature 1), preventative effect on hypertension (Patent literature 2) and its treating agent for insulin-dependent diabetes mettitus (Patent literature 3) have been reported. Also, as other functional characteristics which astaxanthin has, there have been made many reports such as its anti-inflammatory action, action of protecting retina from photic injury, diurnal rhythm adjusting action, immunopotentiation action, anti-stress action, sperm's quality improving action, action of preventing urinary bladder cancer from being induced, action of increasing duration of muscle function, etc.

It has not yet been known that astaxanthin has effects of ameliorating and/or preventing metabolic syndrome by normalizing metabolic abnormalities of mast cell.
[Patent literature 1]: JP 10-155459 A
[Patent literature 2]: JP 2004-534800 A
[Patent literature 3]: JP 2003-510353 A

JP 2003335668 A discloses the antioxidant activity of the claimed compound.

### Disclosure of the invention

### Subject Matter to be Solved by the Invention

As a result of having searched for a composition having an improved and/or preventative effect on metabolic syndrome in order to solve the above object, the present inventors have found that astaxanthin has an effect to ameliorate and/or prevent metabolic syndrome. The present invention has been completed based on such a finding and provides a composition having an effect to ameliorate and/or prevent metabolic syndrome which contains astaxanthin as an effective ingredient as well as a pharmaceutical preparation, beverage and food each having an effect to ameliorate and/or prevent metabolic syndrome which contains astaxanthin as an effective ingredient.

An object of the present invention is to provide a composition having an effect to ameliorate and/or prevent metabolic syndrome which contains astaxanthin as an effective ingredient as well as a pharmaceutical preparation, beverage and food each having an effect to ameliorate and/or prevent metabolic syndrome which each contains astaxanthin as an effective ingredient. The pharmaceutical preparation, beverage and food involving in the present invention have an improved and/or preventative effect on metabolic syndrome whereby improving and/or preventing diseases caused by metabolic syndrome.

### Means for Solving the Subject Matter

As a result of having ardently studied to solve the above object, the present inventors have found that astaxanthin has excellent improved and/or preventative effects on metabolic syndrome. The present invention is based on such a finding.

### Effect of the Invention

By administering in the form of a beverage/food or pharmaceutical preparation the composition having an effect to ameliorate and/or prevent metabolic syndrome which contains astaxanthin as an effective ingredient in the present invention, metabolic syndrome can be improved and/or prevented.

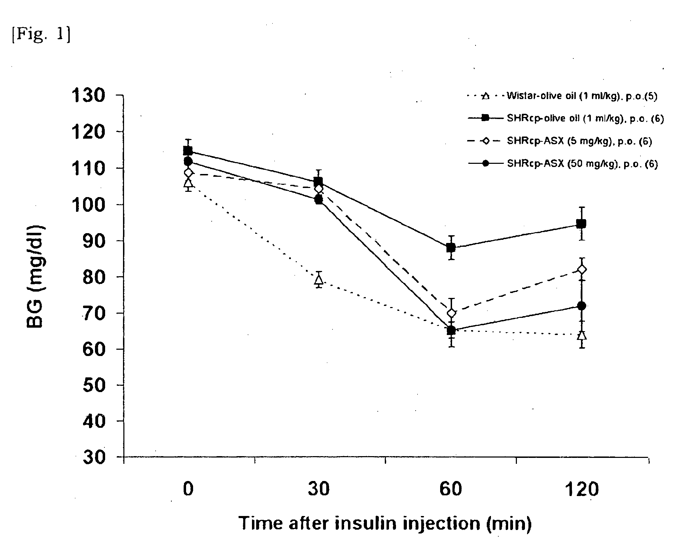

### Brief Explanation of the Drawing

[Fog. 1] is a graph indicating blood glucose concentration after insulin injection in insulin resistance test of a rat of 17 weeks old.

### The Best Mode of Carrying Out the Invention

The term "astaxanthin" in the present invention is meant one derived from natural origin and one obtained by synthesis. As one derived from natural origin, there can be taken crusts, eggs and organs of crustaceans such as shrimp, krill, crab and the like; skins and eggs of various fishes and shellfishes; algae such as green alga of Haematococcus, etc.; yeasts such as *Phaffia* red yeast, etc.; oceanic bacteria; and seed plants such as *Adonis* amurensis and Ranunculus acris. An extract from natural origin and a chemically synthesized product are put on the marketplace and hence they are easily available.

Astaxanthin can be obtained by cultivation of e.g. *Phaffia* red yeast, Hematococcus green alga, oceanic bacteria, etc. in an appropriate medium in accordance with the known method. Hematococcus green alga is the most preferred from the viewpoints of easiness of cultivation and extraction, astaxanthin contained at the highest concentration and high productivity.

As the form to use astaxanthin, the astaxanthin extracts obtained by the before-described processes, powder or aqueous solution each containing them, or dried products of green alga of Haematococcus, of *Phaffia* red yeast, of oceanic bacteria, etc. and pulverized products thereof may be used.

The cultivation method of Hematococcus alga containing astaxanthin and the extraction method of astaxanthin from Hematococcus alga are specifically explained below.

As the cultivation method of Hematococcus green algae, a method cultivating Hematococcus algae using hermetic type of cultivation apparatus is preferable because there is no possibility for different kind of microorganisms to be mixed therein and propagate and because the possibility of other contaminants to be mixed therein is very little. For example, a cultivation process using a partially opened type of dome-like, conical or cylindrical cultivation apparatus wherein culture incuvators are equipped with an optionally movable gas ejector (WO 99/50384 pamphlet). a cultivation process wherein a light source is placed in a hermetic type of cultivation tank and cultivation is conducted under radiation of light from the inner part of the cultivation tank, a cultivation process using a plate-like or tubular cultivation tank, and a method wherein cultivation is conducted under radiation with light having peak wavelength of below about 540 nm (JP 2004-147641 A) are suitable.

As a method of obtaining extract from Hematococcus alga involving in the present invention, Hematococcus alga is pulverized and may be subjected to solvent extraction according to the conventional method. For example, (1) a method wherein after Hematococcus has been pulverized and dried, an extraction is conducted with a solvent and the solvent is removed to obtain an extract and (2) a method wherein after Hematococcus (in wet state) has been suspended in a solvent, the suspension is passed through a pulverizing machine to pulverize a cell and to effect extraction, and then the solvent is removed to obtain an extract are taken. Is preferred the pulverization-extraction method (2) because a contaminant is smallest, the step is short and superfluous heating is not applied to Hematococcus.

The above-described method (1) is one wherein after Hematococcus has been pulverized and dried, an extraction is conducted with a solvent and the solvent is removed to obtain an extract. The pulverization method may be either wet or dry and it may be conducted according to the conventional method such as bead mill, roll mill, hammer mill, jet mill, pin mill, or the like. In this case, the existing antioxidants such as tocopherol, tocotrienol, etc. may be added for preventing an oxidation of astaxanthin. The drying method may be conducted according to the conventional method such as shelf drying, a fluidized bed drying, a flash drying, a spray drying or the like. As a specific pulverization and drying method, it may be conducted according to the conventional method, for example one described in the present applicant's earlier filed WO 2002/077105 pamphlet, Subsequently, the resultant dried and pulverized alga is subjected to a solvent extraction. Example of suitable solvent which can be used for extraction are acetone, alcohol, ethyl acetate, benzene chloroform, carbon dioxide ammonia and the like. The temperature at which an extraction is conducted may be one at which boiling does not occur, and the range of 0 - 60 °C is suitable. Subsequently the resultant extraction liquid is distilled under reduced pressure thereby removing the solvent to obtain an extract.

In case where carbon dioxide or ammonia is used as a solvent for extraction, a superclinical extraction is conducted. The superclinical extraction may be conducted according to the method described in JP 2004-41147 A. The pulverization -treated Hematococcus alga is molded into pellet and the extraction is conducted by passing through a layer filled with the pellet carbon dioxide in superclinical state or in the vicinity of superclinical point. The extract is obtained by removing carbon dioxide under reduced pressure. In order to enhance the extraction efficiency from the pellet, there may be added higher unsaturated fatty acid, glycerin, alcohol water or the like.

The above-described method (2) may be conducted by the method of the present applicant's earlier filed Japanese Patent Application No. 2004-253525. Hematococcus alga in wet state is suspended in such an organic solvent as stated in the above, the suspension is pulverized by a pulverizing machine while extraction is conducted at the same time. The method for removing the organic solvent may be conducted according to the conventional method as stated in the above.

The extract obtained in the above-stated two kinds of methods may be further purified by separation column or lipase decomposition, if desired. For purpose of removing a solvent further, a molecular distillation apparatus or the like may be used.

Since a hermetic type of cultivation apparatus is used, a contaminant derived from different kinds of microorganisms is not present. Also, since an astaxanthin-containing extract is obtained under a mild condition, degradation of the extract by oxidation is small. Accordingly the effect of astaxanthin may be exerted effectively.

Astaxanthin is 3,3'-dihydroxy-β, β-carotene-4, 4'-dione and has stereoisomers. Specifically, such three stereoisomers are known as (3R, 3'R)-astaxanthin, (3R, 3'S)-astaxanthin and (3S, 3'S)-astaxanthin.

Unless otherwise described herein, astaxanthin includes astaxanthin and/or its ester. Furthermore, ester of astaxanthin includes monoester and/or diester.

It is known that astaxanthin has not been observed having any mutagenicity and is highly safe compound and it has been widely used as food additive (Takahashi et al; toxicity test of Hematococcus alga. astaxanthin- Ames test, rat single dose toxicity test, rat 90-days repeat dose oral toxicity test- Journal of Clinical Therapeutic and Medicine, 20:867-881, 2004).

In astaxanthin-containing extract of the present invention, is included at least one of free form, monoester form and diester form of astaxanthin.

The diester form is chemically and physically more stable than the free or monoester form and hard to be subjected to oxidative decomposition in beverage, food or pharmaceutical preparation, because its two hydroxy groups are protected by ester bondage. However, when it is taken into the living body, it is considered quickly hydrolyzed into free astaxanthin by bioenzymes to exert its effect.

As a monoester of astaxanthin, there can be taken monoesters esterified with lower or higher saturated fatty acid, or lower or higher unsaturated fatty acid. Specific examples of lower or higher saturated fatty acid, or lower or higher unsaturated fatty acid include acetic acid, lauric acid, myristic acid, pentadecanoic acid, palmitic acid, palmitoleic acid, heptadecanoic acid, elaidic acid, ricinoleic acid, petroselinic acid, vaccenic acid, eleostearic acid, punicinic acid, licanoic acid, palynalic acid, gadolic acid, 5-eicosenoic acid, 5-docosenoic acid, cetolic acid, ercinoic acid, 5,13-docosadienoic acid, selacholic acid, decenoic acid, stering acid, dodecenoic acid, oleic acid, stearic acid, eicosapentaenoic acid, docosahexaenoic acid, linoleic acid, linolenic acid, arachidonic acid, etc.

As a diester of astaxanthin, there can be taken diesters esterified with the same or different fatty acids selected from the above fatty acids.

Furthermore, as monoester of astaxanthin, there can be taken monoesters esterified with an amino acid such as glycine, alanine or the like; with a mono- or poly-carboxylic acid such as acetic acid, citric acid or the like; with an inorganic acid such as phosphoric acid, sulfuric acid or the like; with a saccharide such as glucoside or the like; with a glyco-fatty acid such as glycoglycero-fatty acid or sphingoglyco-fatty acid; with a fatty acid such as glycero-fatty acid, with glycero-phosphoric acid, etc. In case where it is to be considered, salt of the above monoester is included.

As a diester of astaxanthin, there can be taken diesters esterified with the same or different acids selected from the before-described lower saturated fatty acid, higher saturated fatty acid, lower unsaturated fatty acid, higher unsaturated fatty acid, an amino acid, a mono- or poly-carboxylic acid, an inorganic acid, a sugar, a glyco-fatty acid, fatty acid and glycero-phosphoric acid. In case where it is to be considered, salt of the before-described diesters is included. As a diester of glycero-phosphoric acid, there may be taken saturated fatty acid ester of glycero-phosphoric acid or esters of glycero-phosphoric acid containing fatty acid selected from higher unsaturated fatty acid, unsaturated fatty acid and saturated fatty acid.

The astaxanthin prepared by the extraction method involving in the present invention is particularly effective as compared to the synthetic astaxanthin or one prepared by other extraction method. As one of the causes, it is considered that the extract of the present invention has a good absorbability in the body. As other cause to be considered they are shown below. Since free type of astaxanthin exceeds an intestinal wall, its absorbability into the blood vessel is bad. On the other hand, the astaxanthin derived from a plant such as Hematococcus alga contains a large amount of specific derivatives such as fatty acid. Also, in the case of the extract of the present invention, the oxidations of astaxanthin derivative as the extract and of an unsaturated fatty acid are suppressed during the extraction step thereby the amount of the impurities is small. (Japanese patent Application No. 2004-253525).

In order to enhance the absorbability of astaxanthin into the body and to suppress the release of it, the extract may be formed into powders by adsorbing it on a carrier or by coating-treatment. By a spray drying method, powders may be prepared using a suitable carrier and an emulsifying agent.

The term "metabolic syndrome" in the present invention is meant that by increasing fat in the internal organ, the mast cell therein become obese to cause the change in the normal secretion of physiologically active substances such as TNFα and resistin related to insulin resistance, PAI-1 related to the formation of thrombus, angiotensinogen related to hypertension and the like, and the release of free fatty acid. As a result, two or more of symptoms such as insulin resistance, obesity, hypertriglyceridemia, lowering of HDL cholesterol, hyperpiesia, etc. are combined.

The composition of the present invention is supposed to be effective for the amelioration and the prevention of metabolic syndrome, although the detail of its mechanism is not clear yet, by acting astaxanthin being the main ingredient on mast cells of fat present in the internal organ and bringing the secretion of TNFa, resistin, PAI-1, angiotensinogen and the like, and the release of free fatty acid close to the normal state.

The composition of the present invention has effects of ameliorating and/or preventing metabolic syndrome. Since insulin resistance, obesity, hypertriglyceridemia and hypertension are caused by metabolic syndrome, the composition of the present invention has also an effect of treating, improving and preventing diseases which are mainly caused by these symptoms. Examples of such diseases include arteriosclerosis, hypertension, diabetes, cancer, hyperlipemia, ryeumatism, hyperrucicemia, cerebral accident, ischemic heart disease, pulmonary emphysema, gastric ulcer, gastritis, hepatitis, pancreatitis, nephritis, other inflammatory diseases, cataract, Alzheimer's disease, aging, herpes zoster, complications of diabetes being nerve injury, retinopathy, kidney disease, great vessel injury, and blood disease. In nerve injury, it is effective in treating improving and preventing sudden bradyacusia, abnormality of eye and face (paralysis and pain), depression, dementia, orthostatic hypotension, abnormality of perspiration, flux and obstipation (digestive trouble), dysuria, pain of membrum, pareatrophy, paresthesia, atrophy of muscle, ED, asthenopia, muscle fatigue, contraction of muscle and melancholia. In retinopathy, they are effective in treating improving and preventing macular degeneration, glaucoma, cataract, simple retinosis, preproliferative retinopathy, and proliferative retinopathy. In immune diseases, it is effective in treating improving and preventing autoimmune diseases (allergic disease, vermination, systemic lupus erythematosus, rheumatoid arthritis, Behcet's disease), virus or bacteria infection, malignant tumor (plasmacytoma, multiple myeloma, cancer dyscrasia, atrial myxoma, mycloma, Lennert's lymphoma, etc.), HVC disease or acquired immune deficiency, Kaposi's sarcoma, postclimacteric osteoporosis, inflammatory skin disease (hyperkeratosis, atopic dermatitis, contact dermatitis, etc.), inflammatory bowel diseases (ulcerative colitis, etc. ), inflammatory liver diseases (hepatitis B, hepatitis C, alcoholic heptatitis, etc.), inflammatory kidney diseases (glomerular nephritis etc.) and inflammatory respiratory diseases (asthma, chronic obstructive lung disease, bronchitis, etc.).

The composition of the present invention has not only effect of ameliorating, preventing and treating metabolic syndrome but also effect of ameliorating, preventing and treating diseases caused by metabolic syndrome, and hence it has effect of ameliorating, preventing and treating the before-described diseases caused by metabolic syndrome. The composition of the present invention can be used as a medicine, quasi-drugs, a cosmetic, a functional food, a supplement, a food and a beverage.

The composition of the present invention is one containing astaxanthin. In order to enhance the effect of astaxanthin, one or more of active agents may be added.

Examples of such an active agent include vitamin A substances, carotenoids, vitamin B substances, Vitamin C substances, vitamin D substances, Vitamin E substances, tocotrienol, glutathion, their derivatives and their salts; α-ribo acid, deoxyribonucleic acid, ribonucleic acid, adenosine triphosphate, adenosine monophosphate, glycyrrhizin, glycyrrhizic acid, guanine, xanthine, α- or γ-linolenic acid, eicosapentaenoic acid, succinic acid, estradiol, their derivatives and their salts; aspartic acid, α- hydroxy acid such as glycolic acid, lactic acid, malic acid, citric acid, salicylic acid and the like their derivatives and their salts; an extract of deproteinized serum, spleen extract, placenta extract, crest extract, royal jerry; yeast extract, extract of lactic acid bacteria, extract of bifid bacteria, *Fomes Japonioucus* extract; carrot extract, *Swertia* extract, rosemary extract, phellodendron bark extract, garlic extract, Hinokitiol, cepharanthine, aloe extract, *Salvia splendens* extract, arunica extract, camonile extract, white birch extract, *hypericum* extract, *Eucalyptus* extract, *Xuan Fu Hua* extract, *patholobus suberectus Dunn* extract, Sanpenzu extract, moricortex extract, *Angerica* extract, *Bistorta* extract, *Sophora* extract, *Crataegus* extract, white lily extract, hop extract, wild rose extract, *Coix lacryma-jobi* extract; D-fraction, glycogen, Octacosanol, allicin, coenzyme Q₁₀, catechin; polyphenol, flavinoid, carnosine, ornithine; cystine, its derivative and salt; peptide; lysine, allyl sulfide, biotin, pantothenic acid, collagen, elastin, keratin, their derivatives and salts thereof; hyaluronic acid, chondroitin sulfuric acid, dermatan sulfate, heparitin sulfate, heparin, keratan sulfate; lactic acid bacteria; minerals such as iron, morybdenum, calcium, zinc, selenium, manganese, cupper, iodine and the like; etc. Tocopherol, tocotrienol, α-ribo acid and coenzyme Q₁₀ are preferred.

In case where the composition of the present invention is used as a beverage/food, it may be used in any form including solid, gel and liquid like pharmaceutical preparation or incorporation into the general food. In the case where it is generally used as a functional food, solid form, jelly and drink are preferable. As solid form, it may be used in similar form as pharmaceutical preparation including a tablet, a soft capsule, a hard capsule, granule, powder and the like.

The solid preparation of the composition in the present invention may contain the appropriate amount of various additives which are used for producing general food, especially a functional food or a preparation. Examples of such additives include an excipient, a binding agent, an acidifier, an effervescent agent, an artificial sweetener, a perfume, a lubricant, a coloring agent, a stabilizer, a pH adjusting agent, a surfactant and so on. Examples of an excipient include starches such as corn starch, potato starch, wheat starch, rice starch, partially pregelatinized starch, pregelatinized starch, porous starch and the like; saccharides such as lactose, sucrose, glucose and the like; sugar alcohols such as mannitol, xylitol, erythritol, sorbitol, maltitol and the like; inorganic compounds such as magnesium aluminometasilicate, hydrotalcite, calcium phosphoric acid anhydride, precipitated calcium carbonate, calcium silicate, light anhydrous silicic acid and the like. Examples of a binding agent include hydroxypropyl cellulose, hydroxypropylmethyl cellulose, polyvinylpyrrolidone, gum arabic, gelatin, pullulan, etc. Examples of a disintegrant include starch, agar, starch, methylcellulose, carmellose calcium, sodium carboxymethyl starch, cross carmellose sodium, crospovidone, crystalline cellulose, etc. Examples of an acidifier include citric acid, tartaric acid, malic acid, ascorbic acid and the like. Examples of an effervescent agent include sodium hydrogencarbonate, sodium carbonate and the like. Examples of a sweetener include saccharine sodium, dipotassium glycyrrhizinate, aspartame, stevia, thaumatin, etc. Examples of a perfume include lemon oil, orange oil, menthol, etc. Examples of a lubricant include magnesium stearate, sucrose fatty acid ester, polyethylene glycol, talc, stearic acid, sodium stearyl phthalate, etc. Examples of a coloring agent include food pigments such as food yellow No. 5, food red No. 2, food blue No.2 and the like; food lake pigment, ferric oxide, etc. Examples of a stabilizer include disodium edatate, tocopherol, cyclodextrin, etc. Example of a pH adjusting agent include citrate, phosphate, carbonate, tartrate, fumarate, acetate, salt of amino acid, etc. Examples of a surfactant include polysorbate 80, methyl cellulose, hydroxyethyl cellulose, sodium carboxymethyl cellulose, polyoxyethylene-sorbitan monolaurate, gum arabic, powdered traganth and the like. It is preferable to incorporate them in finely powdered state for making the absorbability of astaxanthin good.

In case where the beverage/food of the present invention is made in a beverage form such as syrup, drink or the like, the effective ingredient may be formed into beverage according to the conventional manner in the presence of a pH adjusting agent, buffering agent, solubilizer, suspension, isotonizing agent, stabilizer, antiseptic and the like depending on necessity. Examples of a suspension include polysorbate 80, methyl cellulose, hydroxyethyl cellulose, sodium carboxymethyl cellulose, polyoxyethylenesorbitan monolaurate, gum arabic, powdered traganth and the like. Examples of a solubilizer include polysorbate 80, polyoxyethylene hydrogenated caster oil, nicotinic acid amide, polyoxyethylenesorbitan monolaurate, macrogol, caster oil fatty acid ethyl ester and the like. Examples of a stabilizer include sodium sulfite, sodium metasulfite and the like. Examples of an antiseptic include methyl p-hydroxy benzoate, ethyl p-hydroxybenzoate, sorbic acid, phenol, cresol, chlorocresol and the like. As other ingredients, there can be incorporated an ingredient, which is generally used in a beverage, such as a mineral, an amino acid and its salts, galenical, its extract, a sweetener, a preservative, a corrigent, a coloring agent or the like.

The beverage involving in the present invention may be prepared according to the conventional manner, and its preparation method is not particularly restricted. Usually, the respective ingredients are mixed with the purified water the amount of which is less than the prescribed amount and then the volume is adjusted to the prescribed amount. If necessary, filtration and sterilization treatment are conducted for the preparation. When oil-soluble vitamin is contained, it may be emulsified or solubilized with a surfactant which is usually used or a solubilizer or otherwise it may be suspended with a dispersing agent.

Astaxanthin may be incorporated into the general beverage/food. As their forms, there may be taken an example of adding it to the general foods such as margarine, butter, butter sauce, cheese, raw cream, shortening, lard, ice cream, yogurt, diary products, meat sauce products, fish products, pickles, fried potato, potato chips, snack confectionery, sliced and dried rice cake, popcorn, a seasoned powder for sprinkling over rice, chewing gum, chocolate, pudding, jelly, gumi-candy, candy, drops, caramel, bread, sponge cake, cake, doughnut, biscuit, cookie, cracker, etc., macaroni, pasta, Chinese noodles, buck wheat, wheat vermicelli, salad oils, instant soup, dressing, egg, mayonnaise, miso, etc., or carbonated or non-carbonated drinks such as fruit drinks, refreshing drinks, sports drinks, etc., non-alcoholic drinks such as tea, coffee, cocoa, etc., or liquors such as liqueur, medical liquor, etc. The beverage and food involving in the present invention may be applied to e.g. a healthy food, a functional food, a nutritional supplement food, a supplement and the like.

The amount incorporated of astaxanthin in the beverage/food involving in the present invention is not particularly restricted and it may be varied depending on the amount of other ingredients to be incorporated thereto. The amount incorporated of astaxanthin may be 0.0001-10 % by weight, preferably 0.0001-5 % by weight based on the total amount of the beverage/food and it is adjusted so as to contain only the amount necessary to exert the preventive or alleviative effect. For application to a functional food, astaxanthin may be contained in an amount of 0.01-99.9 % by weight, preferably 0. 1-90 % by weight based on the total amount of the beverage/food. The amount used of astaxanthin can be selected appropriately depending on the kinds of food and drink by a person having ordinary skill in the art, and it is 0.5-100 mg, preferably 1-20 mg per day for an adult.

In the case where both of astaxanthin and an active agent are incorporated in the beverage/food of the present invention, they may be incorporated in a ratio of 0.1-10 parts by weight, preferably 0.5-5 parts by weight of an active agent to 1 part by weight of astaxanthin. The total content of astaxanthin and an active agent in the beverage/food of the present invention may be 0.00001-99.9 % by weight, preferably 0.001-90 % by weight. For application to a functional food, the total content of astaxanthin and an active agent may be 0.01-99.9 % by weight, preferably 0. 1-90 % by weight based on the total amount of the beverage/food. A person having ordinary skill in the art can select the amount used of the active agent appropriately depending on the kinds of composition and the amount of intake per day for an adult.

The pharmaceutical preparation of the present invention which has an improved effect on metabolic syndrome may be administered orally or parenterally. As oral dosing preparation, it may be administered in solid dosing forms such as tablet, intraoral disinteegratale tablet, capsule, granule, powder and the like and in liquid dosing forms such as syrup and suspension and the like. As parenteral dosing preparation, it may be administered in the forms of parenteral, eye drops, nose drops, patch, paste and suppository. The lipid-dispersed type of preparation is effective for increasing the concentration in blood.

The pharmaceutical preparation of the present invention may be made into solid preparation by incorporating various kinds of ingredients which may be used in the before-described solid preparation for the beverage/food. A liquid preparation such as syrup, drink, suspension, eye drops, parenteral or the like may be made by incorporating various kinds of ingredients which may be used in a beverage of the before-described beverage/food.

Also, for a skin external application agent, in addition to the above-described ingredients, there may be adequately incorporated ingredients which are usually used for cosmetics and skin external application agents such as medicament and the like depending on the necessity. Example of such ingredients include a whitening agent, a humectant, an antioxidant, an oil component, an ultraviolet absorber, a surfactant, a viscosity-increasing agent, alcohols, a powder component, a coloring agent, an aqueous component, water, various skin nutrients, etc.

The amount used of astaxanthin in the pharmaceutical preparation of the present invention is 0.5-100mg, preferably 1-20 mg in terms of free astaxanthin per day for adult, and an oral or parenteral administration is conducted in such dose. The dosage may vary in age, body weight or grade of symptoms of a patient to be administered and the dosing form. The astaxanthin content in the pharmaceutical preparation of the present invention may be 0.01-99.9 % by weight, preferably 0.1-90 % by weight.

In the case where both of astaxanthin and an active agent are incorporated in the pharmaceutical preparation of the present invention, they may be incorporated in a ratio of 0.1-20 parts by weight, preferably 0.5-10 parts by weight of the active agent to 1 part by weight of astaxanthin. The total content of astaxanthin and the active agent in the pharmaceutical preparation of the present invention may be 0.01-99.9 % by weight, preferably 0.1-90 % by weight.

### Example 1

### [Measurement of blood pressure]

Rats were broken in the apparatus since a week ago from the beginning of the experiment. They were bound slightly under a non-anesthetic and cuff with a built-in infrared sensor (THC-1, a product of Softron Co., Ltd. was applied to the tail artery to measure pressure (maximum, minimum and an average blood pressures) by a plethysmography. The measurement of blood pressure was conducted in the afternoon.

### [Measurement of blood glucose value]

Water only was given to rats ad lib from the day before the measurement not so as to give any feed to them. In the afternoon, 0.5 U of insulin was injected to them in an amount of 1 ml per 1 kg of the body weight, and the exsanguinations were conducted at given times to measure the glucose concentration in blood using a Antisence n (a product of Horive Ltd).

### [Measurement of fat in blood]

The supernatant formed after centrifugation was subjected to the triglyceride G test using an enzyme (a product of Wako Pure Chemical Industries, Ltd.) to measure the fat concentration in blood.

### [Measurement of adiponectin in blood]

I ml of blood was exsanguinated from a rat. After centrifugation, the obtained supernatant was subjected to ELISA kit for measuring adiponectin (Otsuka Pharmaceutical Inc.) to measure the adiponectin concentration in blood

### [Preparation of Hematococcus alga extract]

1 Kg of dried and unpulverized Hematococcus alga powder (a product of Bio-Real Inc.), astaxanthin content of 4.6 %) and 25 g of mixed vitamins (a product of Riken Vitamin, E700) were dispersed in 1.5 kg of acetone, and the resultant dispersion was subjected twice to pulverization treatment a using anti-explosive type of bead mill tester [DYDO-MILL KDL-PILOT, a product of WAB company] wherein bead mill having a diameter of 1 mm was compacted at a filling rate of 85 % at room temperature cooling when required under disc peripheral speed of 10 m/s and a flow velocity of 140 g/min to extract a lipid fraction containing carotenoid at the same time.

The total amount of crushed suspension was subjected to sunction filtration to recover an extraction filtrate. Furthermore, extraction residue was rinsed three times with 1 kg of acetone to extract lipid ingredients containing carotenoid completely. Acetone was removed from the extraction filtrate obtained with a vacuum evaporator (at a temperature of 35 °C) and a very small amount of acetone was further remove a molecular distillation apparatus to obtain the astaxanthin- containing extract (content of 5.6 % in terms of the free form).

### [Experimental Example]

Female 5 weeks old, metabolic syndrome model rats (SHR/Ndmer) (a product of Sankyo laboratories Co., Ltd.) were purchased and reared for about a week in a laboratory animals rearing room of a constant temperature (24 ± 1 °C), a constant humidity (54 ± 5 %) and a 12 hour light-dark cycle, and thereafter used for the experiment. Water and feed were provided ad lib to them. In the experiment, each group consists of 6 rats with proviso that 5 Wistar rats as normal group were used. Sample was orally administered to all rats in the afternoon.

### <Administration groups>

1) Control group: olive oil was administered in an amount of 1 ml per 1 kg of body weight of rat.
2) Astaxanthin 5 mg/Kg administration group: astaxanthin was administered in an amount of 5 mg in admixture with I ml of olive oil per 1 kg of body weight of rat.
3) Astaxanthin 50 mg/Kg administration group: astaxanthin was administered in an amount of 50 mg in admixture with I ml of olive oil per 1 kg of body weight of rat.
4) Normal group: olive oil was administered in an amount of 1 ml per 1 kg of body weight of Wister rat

In tables 1-7 shown below, the term "Ax (5 mg/kg)" indicates astaxanthin 5 mg/Kg administration group and the term "Ax (50 mg/kg)" does astaxanthin 50 mg/Kg administration group.

**[Table 1] Change in body weight**

| Sample | At 6 weeks old | At 9 weeks old | At 13 weeks old | At 17 weeks old |
|---|---|---|---|---|
| Normal Group | 159 | 248 | 337 | 390 |
| Control Group | 154 | 255 | 362 | 456 |
| Ax (5mg/kg) | 156 | 259 | 378 | 468 |
| Ax (50mg/kg) | 148 | 250 | 368 | 459 |

| | | | | |
|---|---|---|---|---|
| A: p<0.05 (t-test, control group vs 5mg/kg, 50mg/kg), Unit: g | | | | |

It can be seen that there is no change in the body weight by the administration of astaxanthin.

**[Table 2] Change in diastolic blood pressure**

| Sample | At 6 weeks old | At 9 weeks old | At 12 weeks old | At 24 weeks old |
|---|---|---|---|---|
| Control Group | 122 | 132 | 144 | 179 |
| Ax (5mg/kg) | 121 | 130 | 129 | 155 |
| Ax (50mg/kg) | 121 | 128 | 119 | 156 |

| | | | | |
|---|---|---|---|---|
| A: p<0.05 (control group vs 5mg/kg, 50mg/kg), Unit: mmHG | | | | |

**[Table 3] Change in systolic blood pressure**

| Sample | At 6 weeks old | At 9 weeks old | At 12 weeks old | At 24 weeks old |
|---|---|---|---|---|
| Control Group | 148 | 162 | 173 | 208 |
| Ax (5mg/kg) | 144 | 161 | 159 | 185 |
| Ax (50mg/kg) | 147 | 160 | 154 | 189 |

| | | | | |
|---|---|---|---|---|
| A: p<0.05 (t-test, control group vs 5mg/kg, 50mg/kg), Unit: mmHG | | | | |

It can be seen that the administration of astaxanthin-containing beverage/food of the present invention suppresses the rise in blood pressure.

**[Table 4] Change in fasting blood glucose value**

| Sample | At 7 weeks old | At 9 weeks old | At 12 weeks old | At 17 weeks old |
|---|---|---|---|---|
| Normal Group | 137.0 | 86.3 | 95.3 | 106.0 |
| Control Group | 138.3 | 96.2 | 91.7 | 114.6 |
| Ax (5mg/kg) | 132.0 | 98.0 | 88.3 | 108.8 |
| Ax (50mg/kg) | 135.0 | 109.0 | 89.3 | 111.8 |

| | | | | |
|---|---|---|---|---|
| A: p<0.01 (t-test, control group vs 5mg/kg, 50mg/kg), Unit: mg/dl. Rats were placed under fasting state an overnight (while water was provided ad lib to them) to measure the glucose concentration in blood. | | | | |

**[Table 5] Change in blood glucose value after insulin infection**

| Sample | At 7 weeks old | At 9 weeks old | At 12 weeks old | At 17 weeks old |
|---|---|---|---|---|
| Normal Group | 54.0 | 47.8 | 51.0 | 64.0 |
| Control Group | 107.7 | 87.8 | 88.0 | 94.7 |
| Ax (5mg/kg) | 98.0 | 85.6 | 81.5 | 82.2 |
| Ax (50mg/kg) | 106.3 | 81.0 | 73.8 | 72.0 |

| | | | | |
|---|---|---|---|---|
| A: p<0.05 (t-test, control group vs 5mg/kg, 50mg/kg), Unit: mg/dl. Rats were placed under fasting state an overnight (while water was provided ad lib to them). 0.5U of insulin was injected to them in an amount of 1 ml per kg of body weight and the glucose concentration in blood after 120 minutes was measured. | | | | |

It can be seen that the administration of astaxanthin-containing beverage/food of the present invention decreases the glucose concentration in blood at the time when insulin was injected. The insulin resistance was significantly improved.

**[Table 6] Change in blood fat**

| Sample | At 7 weeks old | At 12 weeks old |
|---|---|---|
| Normal Group | 52.5 ± 2.1 | 25.4 ± 2.7 |
| Control Group | 162.1 ± 5.6 | 414.8 ± 9.5 |
| Ax (50mg/kg) | 139.5 ± 15.9 | 304.8 ± 9.9 |

| | | |
|---|---|---|
| A: p<0.001 (t-test, control group vs 50mg/kg), Unit: mg/dl | | |

The astaxanthin-administration group has less blood fat in comparison with the control group. Therefore, astaxanthin suppresses the increase in the blood fat in SHR/Ndmer with the passage of time.

**[Table 7] Change in blood adiponectin concentration**

| Sample | At 7 weeks old | At 12 weeks old |
|---|---|---|
| Normal Group | 4.81 ± 0.28 | 4.44 ± 0.59 |
| Control Group | 7.38 ± 0.27 | 5.91 ± 0.16 |
| Ax (50mg/kg) | 7.08 ± 0.43 | 8.21 ± 0.14 |

| | | |
|---|---|---|
| A: p<0.01 (t-test, control group vs 50mg/kg), Unit: mg/ml | | |

It can be seen that the adiponectin concentration in blood is increased in comparison with the control group by the administration of astaxanthin. Since adiponectin has an action of promoting the absorption of glucose into cell and the combustion of fat, astaxanthin is considered to promote the increase in blood adiponectin and to participate in the decrease in the blood glucose and fat.

### [Preparation Example 1] Tablet

The following ingredients were uniformly mixed together in the composition ratio(% by weight) to prepare tablets, each having 300 mg of weight.

| | |
|---|---|
| Hematococcus alga extract | 30 mg |
| Lactose | 70 mg |
| Starch | 70 mg |
| Sodium caseinate | 6 mg |
| Gelatin | 6 mg |
| Cellulose | 109 mg |
| Silicon dioxide | 3 mg |
| Sucrose fatty acid ester | 6 mg |

Hematococcus alga extract contains 5 % by weight of astaxanthin in terms of free form.

### [Preparation Example 2] Soft capsule

Hematococcus alga extract (astaxanthin content of 5 % by weight) was filled in the outer shell of capsule consisting of the following ingredients according to the conventional method to make soft capsules, each having 300 mg of weight.

| Inner filling | |
|---|---|
| Hematococcus alga extract | 20 mg |
| Eatable oil and fat | 150 mg |

| Outer shell | |
|---|---|
| Gelatin | 100mg |
| Glycerin | 30 mg |

### [Preparation Example 3] Drink

The following ingredients were compounded together and 10 kg of water was added thereto according to the conventional method to prepare a drink.

| | |
|---|---|
| An aqueous solution of Hematococcus alga extract* | 25 g |
| Liquid sugar | 4000 g |
| Sodium DL-tartrate | 1g |
| Citric acid | 50 g |
| Vitamin C | 50 g |
| Vitamin E | 150 g |
| Cyclodextrin | 25 g |
| Potassium chloride | 5g |
| Magnesium sulfate | 2g |

| | |
|---|---|
| *An aqueous solution of Hematococcus alga extract (astaxanthin content of 1 %) prepared by the method of example described in JP 2001-316601 A | |

### [Preparation Example 4] Stick granule

The following ingredients were compounded and granulated according to the conventional method to prepare stick granules, each containing 5 g.

| | |
|---|---|
| Astareal powder* | 5 % |
| Vitamin B mix | 1 % |
| Nicotinic acid | 0.1 % |
| Panthothenic acid | 0.1 % |
| Taurine | 10 % |
| Glutamic | 1 % |
| GABA | 0.01 % |
| Aspartic acid | 0.05 % |
| BCAA | 0.5 % |
| Citric acid | 10 % |
| Vitamin C | 10 % |
| γ-Oryzanol powder | 0.15 % |
| CMCNa | Suitable amount |
| Dextrin | Suitable amount |

| | |
|---|---|
| *Powder of Hematococcus alga extract containing 1 % by weight of astaxanthin | |

### [Preparation Example 5] Chocolate

The following ingredients were compounded and chocolate was prepared according to the conventional method.

| | |
|---|---|
| Astareal oil 50F | 1% |
| Propolis | 0.1% |
| Royal jelly | 0.01% |
| Caffeine | 0.5% |
| Extract of carrot | 0.5% |
| Lecithin | Suitable amount |
| Cacao mass | Suitable amount |

## Claims

1. A composition which contains astaxanthin as an effective ingredient for use in a method of ameliorating and/or preventing metabolic syndrome.

2. A composition according to claim 1 for the use according to claim 1, wherein the composition contains astaxanthin and one or more active agents as an effective ingredient.

3. The composition according to claims 1-2 for the use according to claim 1 wherein the astaxanthin is one obtained by pulverizing *Haematococcus* algae, subjecting pulverized *Haematococcus* algae to solvent extraction and removing the solvent from the extract.

4. The composition according to claims 2-3 for the use according to claim 1 wherein the active agent is one or more of substances in any one of the following groups:
vitamin A substances, carotenoids, vitamin B substances, Vitamin C substances, vitamin D substances, Vitamin E substances, tocotrienol, glutathion, their derivatives and their salts; α-ribo acid, deoxyribonucleic acid, ribonucleic acid, adenosine triphosphate, adenosine monophosphate, glycyrrhizin, glycyrrhizic acid, guanine, xanthine, α- or γ-linolenic acid, eicosapentaenoic acid, succinic acid, estradiol, their derivatives and their salts; aspartic acid, α- hydroxy acid such as glycolic acid, lactic acid, malic acid, citric acid, salicylic acid and the like their derivatives and their salts; an extract of deproteinized serum, spleen extract, placenta extract, crest extract, royal jelly; yeast extract, extract of lactic acid bacteria, extract of bifid bacteria, *Fomes Japonioucus* extract; carrot extract, *Swertia* extract, rosemary extract, phellodendron bark extract, garlic extract, Hinokitiol, cepharanthine, aloe extract, *Salvia splendens* extract, arunica extract, camomile extract, white birch extract, *hypericum* extract, *Eucalyptus* extract, *Xuan Fu Hua* extract, *patholobus suberectus Dunn* extract, Sanpenzu extract, moricortex extract, *Angerica* extract, *Bistorta* extract, *Sophora* extract, *Crataegus* extract, white lily extract, hop extract, wild rose extract, *Coix lacryma-jobi* extract; D-fraction, glycogen, Octacosanol, allicin, coenzyme Q₁₀, catechin; polyphenol, flavinoid, carnosine, ornithine; cystine, its derivative and salt; peptide; lysine, allyl sulfide, biotin, pantothenic acid, collagen, elastin, keratin, their derivatives and salts thereof; hyaluronic acid, chondroitin sulfuric acid, dermatan sulfate, heparitin sulfate, heparin, keratan sulfate; lactic acid bacteria; minerals such as iron, molybdenum, calcium, zinc, selenium, manganese, copper, and iodine.

5. The composition according to claims 1-4 for the use according to claim 1, wherein the composition is a pharmaceutical preparation.

6. The composition according to claims 1-4 for the use according to claim 1, wherein the composition is a beverage or a food.

## Patentansprüche

1. Zusammensetzung enthaltend Astaxanthin als einen wirksamen Bestandteil zur Verwendung in einem Verfahren zur Linderung und/oder Prävention von metabolischem Syndrom.

2. Zusammensetzung nach Anspruch 1 zur Verwendung nach Anspruch 1, wobei die Zusammensetzung Astaxanthin und ein oder mehr aktive Mittel als aktiven Bestandteil enthält.

3. Zusammensetzung nach den Ansprüchen 1-2 zur Verwendung nach Anspruch 1, wobei das Astaxanthin erhalten worden ist durch Pulverisieren von *Haematococcus*-Algen, Unterwerfen pulverisierter *Haematococcus*-Algen einer Lösungsmittelextraktion und Entfernen des Lösungsmittels aus dem Extrakt.

4. Zusammensetzung nach den Ansprüchen 2-3 zur Verwendung nach Anspruch 1, wobei das aktive Mittel eine oder mehrere Substanzen aus einer der folgenden Gruppen ist:
Vitamin-A-Substanzen, Carotinoide, Vitamin-B-Substanzen, Vitamin-C-Substanzen, Vitamin-D-Substanzen, Vitamin-E-Substanzen, Tocotrienol, Glutathion, deren Derivate und deren Salze; α-Ribosäure, Deoxyribonucleinsäure, Ribonucleinsäure, Adenosintriphosphat, Adenosinmonophosphat, Glycyrrhizin, Glycyrrhizinsäure, Guanin, Xanthin, α-oder γ-Linolensäure, Eicosapentaensäure, Bernsteinsäure, Estradiol, deren Derivate und deren Salze; Asparaginsäure, α-Hydroxysäure, wie z.B. Glycolsäure, Milchsäure, Äpfelsäure, Citronensäure, Salicylsäure und dergleichen, deren Derivate und deren Salze; ein Extrakt aus deproteiniertem Serum, Milzextrakt, Plazentaextrakt, Crest-Extrakt, Gelee royal; Hefeextrakt, Extrakt aus Milchsäurebakterien, Extrakt aus Bifidus-Bakterien, *Fomes-Japonioucus*-Extrakt; Karottenextrakt, *Swertia*-Extrakt, Rosmarinextrakt, Phellodendronrindenextrakt, Knoblauchextrakt, Hinokitiol, Cepharanthin, Aloeextrakt, *Salvia*-*splendens*-Extrakt, Arunica-Extract, Kamillenextrakt, Weißer-Birken-Extrakt, *Hypericum*-Extrakt, *Eucalyptus*-Extrakt, *Xuan-Fu-Hua*-Extrakt, *Patholobus-suberectus-Dunn*-Extrakt, Sanpenzu-Extrakt, Moricortex-Extrakt, *Angerica*-Extrakt, *Bistorta*-Extrakt, *Sophora*-Extrakt, *Crataegus*-Extrakt, Weißer-Lilien-Extrakt, Hopfenextrakt, Wildrosenextrakt, *Coix-lacryma-jobi*-Extrakt; D-Fraktion, Glycogen, Octacosanol, Allicin, Coenzym Q₁₀, Catechin; Polyphenol, Flavinoid, Carnosin, Ornithin; Cystin, dessen Derivate und Salze; Peptid; Lysin, Allylsulfid, Biotin, Pantothensäure, Collagen, Elastin, Keratin, deren Derivate und deren Salze; Hyaluronsäure, Chondroitinschwefelsäure, Dermatansulfat, Heparitinsulfat, Heparin, Keratansulfat; Milchsäurebakterien; Mineralstoffe, wie z.B. Eisen, Molybdän, Calcium, Zink, Selen, Mangan, Kupfer und lod.

5. Zusammensetzung nach den Ansprüchen 1-4 zur Verwendung nach Anspruch 1, wobei die Zusammensetzung ein pharmazeutisches Präparat ist.

6. Zusammensetzung nach den Ansprüchen 1-4 zur Verwendung nach Anspruch 1, wobei die Zusammensetzung ein Getränk oder ein Nahrungsmittel ist.

## Revendications

1. Composition qui contient de l'astaxanthine en tant qu'ingrédient efficace destinée à une utilisation dans un procédé d'amélioration et/ou de prévention d'un syndrome métabolique.

2. Composition selon la revendication 1 destinée à une utilisation selon la revendication 1, dans laquelle la composition contient de l'astaxanthine et un ou plusieurs agents actifs en tant qu'ingrédient efficace.

3. Composition selon les revendications 1 et 2 destinée à une utilisation selon la revendication 1, dans laquelle l'astaxanthine est obtenue par pulvérisation d'algues *Haematococcus,* par soumission des algues *Haematococcus* pulvérisées à une extraction au solvant et l'élimination du solvant de l'extrait.

4. Composition selon les revendications 2 et 3 destinée à une utilisation selon la revendication 1, dans laquelle l'agent actif est une ou plusieurs substances de l'un quelconque des groupes suivants :
les substances à base de vitamine A, les caroténoïdes, les substances à base de vitamine B, les substances à base de vitamine C, les substances à base de vitamine D, les substances à base de vitamine E, le tocotriénol, le glutathion, leurs dérivés et leurs sels ; l'acide α-riboïque, l'acide désoxyribonucléique, l'acide ribonucléique, l'adénosine triphosphate, l'adénosine monophosphate, la glycyrrhizine, l'acide glycyrrhizique, la guanine, la xanthine, l'acide α ou γ-linolénique, l'acide éicosapentaénoïque, l'acide succinique, l'estradiol, leurs dérivés et leurs sels ; l'acide aspartique, un α-hydroxyacide tel que l'acide glycolique, l'acide lactique, l'acide malique, l'acide citrique, l'acide salicylique, et analogues, et leurs dérivés et leurs sels ; un extrait de sérum déprotéinisé, l'extrait de rate, l'extrait de placenta, l'extrait de crête, la gelée royale ; l'extrait de levure, l'extrait de bactéries d'acide lactique, l'extrait de bactéries de bifide, l'extrait de *Formes Japonioucus ;* l'extrait de carotte, l'extrait de *Swertia,* l'extrait de romarin, l'extrait d'écorce de phellodendron, l'extrait d'ail, l'Hinokitiol, la cépharanthine, l'extrait d'aloes, l'extrait de *Salvia splendens,* l'extrait d'arunica, l'extrait de camomille, l'extrait de bouleau blanc, l'extrait d'hypericum, l'extrait d'Eucalyptus, l'extrait de *Xuan Fu Hua,* l'extrait de *patholobus suberectus Dunn,* l'extrait de Sanpenzu, l'extrait de moricortex, l'extrait d'*Angerica,* l'extrait de *Bistorta,* l'extrait de *Sophora,* l'extrait de *Crataegus,* l'extrait de lis blanc, l'extrait d'hop, l'extrait de rose sauvage, l'extrait de *Coix lacrymajobi ;* la D-fraction, le glycogène; l'octacosanol, l'allicine, la coenzyme Q₁₀, la cathéchine ; le polyphénol, le flavinoïde, la carnosine, l'ornithine ; la cystéine, son dérivé et son sel ; un peptide ; la lysine, le sulfure d'allyle, la biotine, l'acide pantothénique, le collagène, l'élastine, la kératine, leurs dérivés et leurs sels ; l'acide hyaluronique, l'acide chondroïtine-sulfurique, le sulfate de dermatan, le sulfate d'héparine, l'héparine, le sulfate de kératan ; les bactéries d'acide lactique ; des minéraux tels que le fer, le molybdène, le calcium, le zinc, le sélénium, le manganèse, le cuivre et l'iode.

5. Composition selon les revendications 1 à 4 destinée à une utilisation selon la revendication 1, dans laquelle la composition est une préparation pharmaceutique.

6. Composition selon les revendications 1 à 4 destinée à une utilisation selon la revendication 1, dans laquelle la composition est une boisson ou un aliment.
